Europäisches Patentamt

European Patent Office

Office européen des brevets

(19)

(11) Numéro de publication: **0 119 148**

**A1**

(12) **DEMANDE DE BREVET EUROPEEN**

(21) Numéro de dépôt: **84460001.5**

(22) Date de dépôt: **23.02.84**

(51) Int. Cl.³: **A 61 N 1/40**

(30) Priorité: **04.03.83 FR 8303969**

(43) Date de publication de la demande:
**19.09.84 Bulletin 84/38**

(84) Etats contractants désignés:
**AT BE CH DE GB IT LI LU NL SE**

(71) Demandeur: **Compagnie Française d'Electronique
Médicale International S.A. ( C.O.F.R.E.M. International
SA)
La Brosse
F-35760 Saint-Gregoire(FR)**

(72) Inventeur: **Renault, Joel Emmanuel
2, rue du Champ Sévigné
F-35760 Saint-Gregoire(FR)**

(74) Mandataire: **Le Guen, Louis François
13, rue Emile Bara BP 91
F-35802 Dinard Cedex(FR)**

(54) Appareil thérapeutique athermique.

(57) L'appareil comprenant une partie analogique de génération et de mise en forme du signal haute fréquence émis par
la tête de traitement et une partie logique de génération de
signaux de découpage temporel et de réglage d'amplitude
du signal haute fréquence.

La partie analogique comprend, montés en cascade, un
oscillateur haute fréquence (1) stabilisé par un quartz (X), un
étage de modulation (2), un étage de préamplification (3), un
étage d'amplification de puissance (4) et un filtre de bande
(5) dont la sortie est reliée à l'entrée d'excitation de la tête (6).
La partie logique (7) délivre un train d'impulsions appliquées
à l'entrée de modulation du modulateur (2) et comprend un
circuit numérique de réglage (10) de la durée des impulsions,
un circuit de réglage (9) de la fréquence des impulsions, un
circuit de réglage (11) de l'amplitude des impulsions et un
circuit de réglage (8) de la durée continue ou cadencée du
fonctionnement.

FIG.1

"Appareil thérapeutique athermique"

La présente invention concerne un appareil thérapeutique athermique et, plus particulièrement, les circuits électroniques qui le composent.

Les appareils thérapeutiques athermiques sont connus depuis une quarantaine d'années. A titre d'illustration, on pourra se reporter aux descriptions d'appareils de ce genre décrits dans les brevets US - 2 448 541, US - 3 503 403, US - 3 515 851, FR - 2 097 138, FR - 2 291 773, FR - 2 301 965.

Tous les appareils thérapeutiques athermiques comprennent un oscillateur haute fréquence alimentant une entrée de signal d'un modulateur dont l'entrée de commande est reliée à un générateur d'impulsions et dont la sortie est reliée à une tête de traitement, par un transformateur. Des amplificateurs sont connectés dans ce circuit pour obtenir les niveaux adéquats.

A l'origine, l'oscillateur, le modulateur et le générateur d'impulsions étaient des circuits électroniques à lampes. Puis, au fur et à mesure de l'évolution de la technique, ces circuits ont été progressivement transistorisés, à l'exception des étages de puissance dans lesquels des lampes étaient conservées. Il fallait donc, dans

2                                                          0119148

ces appareils connus, prévoir deux sources d'alimentation.

Un objet de l'invention consiste à prévoir un appareil thérapeutique entièrement transistorisé.

Suivant une caractéristique de l'invention, il est prévu un appareil comprenant une partie analogique de génération et de mise en forme du signal haute fréquence émis par la tête de traitement et une partie logique de génération de signaux de découpage temporel et de réglage d'amplitude de crête du signal haute fréquence, la partie analogique comprenant, montés en cascade, un oscillateur haute fréquence stabilisé par un quartz, un étage de modulation, un étage de préamplification, un étage d'amplification de puissance et un filtre de bande dont la sortie est reliée à l'entrée d'excitation de la tête, la partie logique délivrant un train d'impulsions appliquées à l'entrée de modulation du modulateur et comprenant un circuit numérique de réglage de la durée des impulsions, un circuit numérique de réglage de la fréquence des impulsions, un circuit de réglage de l'amplitude des impulsions et un circuit de réglage de la durée continue ou cadencée du fonctionnement de l'appareil.

Suivant une autre caractéristique, le modulateur est un amplificateur à transistor fonctionnant en classe C et accordé sur la fréquence de l'oscillateur haute fréquence, la tension d'alimentation de l'amplificateur étant déterminée par le signal de sortie de la partie logique qui lui applique le train d'impulsions.

Les caractéristiques de l'invention mentionnées ci-dessus, ainsi que d'autres, apparaîtront plus clairement à la lecture de la description d'un exemple de réalisation, ladite description étant faite en relation avec les dessins joints, parmi lesquels:

la Fig. 1 est un un bloc-diagramme d'un appareil thérapeutique,

la Fig. 2 est le schéma de l'oscillateur haute fréquence,

la Fig. 3 est le schéma du modulateur,

la Fig. 4 est le schéma du préamplificateur,

la Fig. 5 est le schéma de l'amplificateur de puissance,

la Fig. 6 est le schéma du circuit de réglage des largeurs des impulsions,

la Fig. 7 est le schéma du circuit de réglage de la fréquence des impulsions,

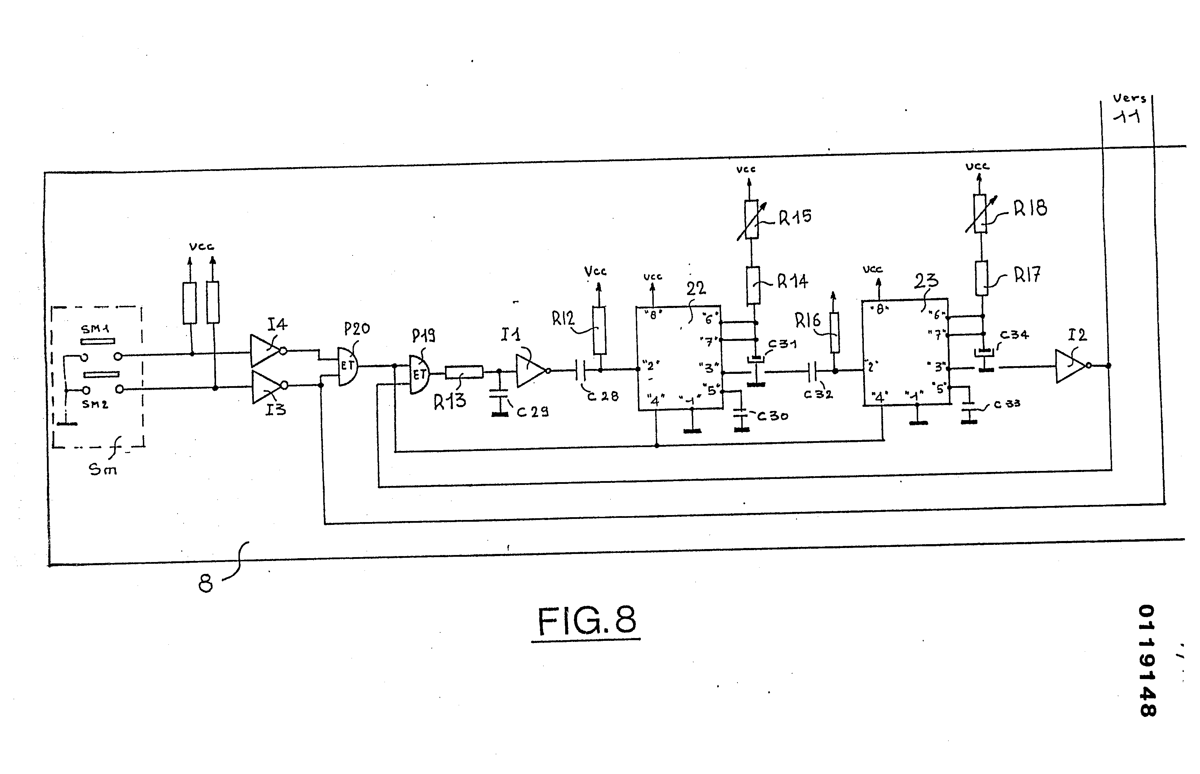

la Fig. 8 est le schéma du circuit de réglage des durées de salves d'impulsions,

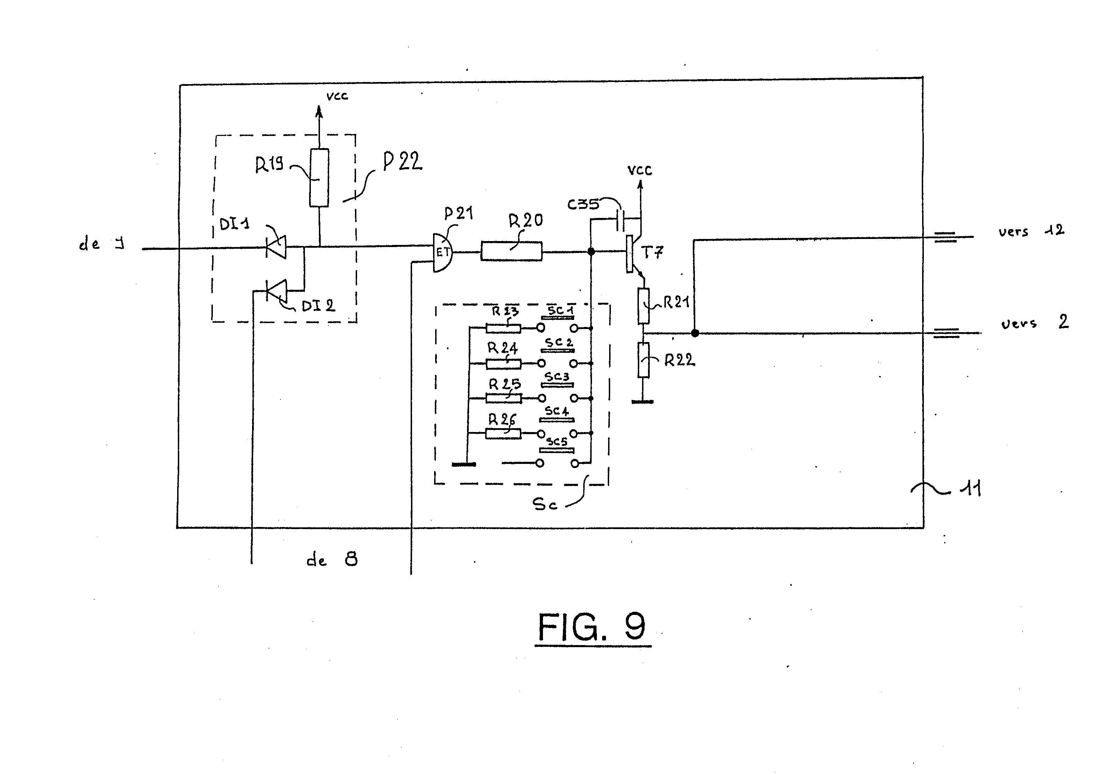

la Fig. 9 est le schéma du circuit de synthèse de la modulation par impulsions et de réglage de la puissance de crête des impulsions,

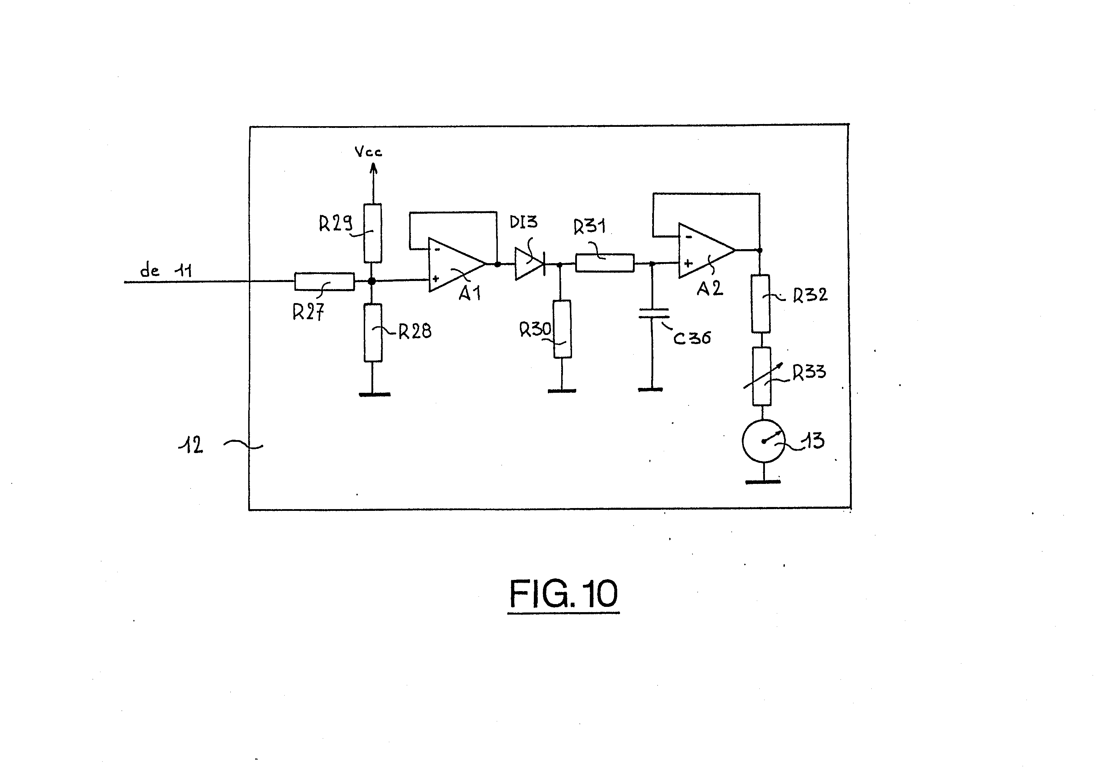

la Fig. 10 est le schéma du circuit de calcul de la puissance moyenne,

les Figs. 11a et 11b sont des diagrammes illustrant le fonctionnement du circuit 8, et

la Fig. 12 est un diagramme illustrant le fonctionnement des circuits des Figs. 6 et 7.

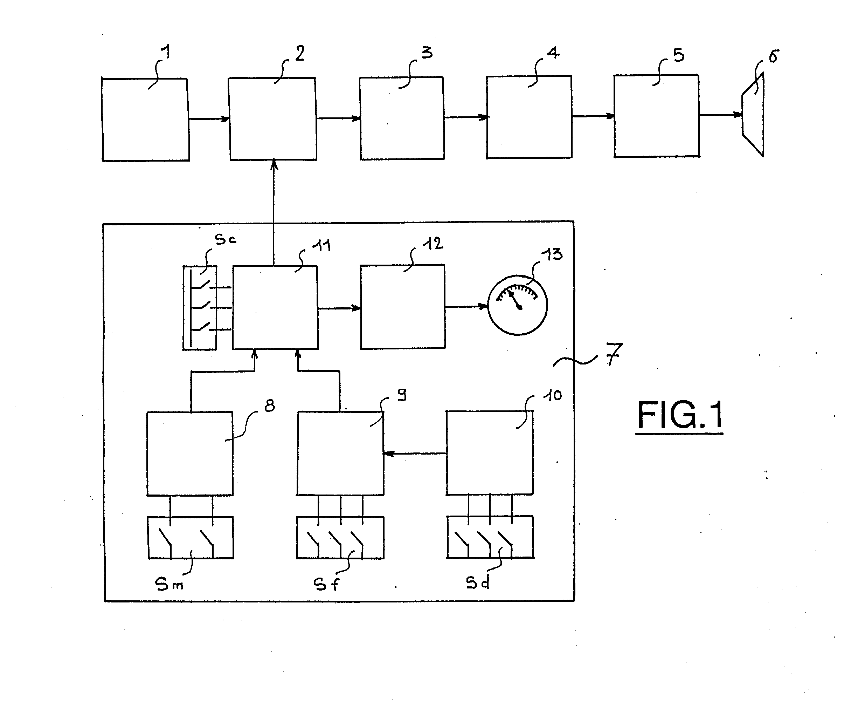

L'appareil thérapeutique de la Fig. 1 se compose d'une partie analogique de génération et de traitement du signal haute fréquence et d'une partie logique de génération de signaux de découpage temporel et de réglage d'amplitude.

La partie analogique haute fréquence comprend un oscillateur 1 dont la sortie est reliée à l'entrée haute fréquence d'un modulateur 2 dont la sortie de signal modulé est reliée à l'entrée d'un préamplificateur 3 dont la sortie est reliée à l'entrée d'un amplificateur de puissance 4 dont la sortie est reliée à l'entrée d'un filtre 5 dont la sortie est reliée à une tête de traitement 6. L'entrée de modulation du modulateur 2 est reliée à la sortie d'un générateur de signal modulant 7.

Le générateur 7, qui constitue la partie logique de l'appareil, comprend un circuit de réglage de temps de fonctionnement 8 réglable au moyen de touches de commandes Sm dont la sortie est reliée, par une liaison 14, à une entrée d'un circuit de synthèse de modulation 11. Le générateur 7 comprend encore un générateur d'impulsions 10 associé à un jeu de touches Sd permettant de régler la largeur des impulsions. La sortie du générateur d'impulsions 10 est reliée à l'entrée de modulation d'un oscillateur modulable 9 dont la fréquence est réglable au moyen d'un jeu de touches Sf. La sortie pulsée de l'oscillateur 9 est reliée, par une liaison 15 à une seconde entrée du circuit de synthèse 11. Le circuit de synthèse 11 est associé à un jeu de touches Sc et a sa sortie reliée, d'une part, à l'entrée de modulation du modulateur 2 et, d'autre part, à un calculateur analogique de moyenne 12 dont la sortie est reliée à un instrument de mesure 13 du type milliampèremètre.

L'oscillateur 1 est un oscillateur à transistor à fréquence stabilisée par un quartz. Le quartz a été choisi de manière que la fréquence de fonctionnement soit égale, dans l'exemple de réalisation, à 27,125 MHz, c'est à dire dans la bande HF. L'oscillateur proprement dit est suivi d'un premier amplificateur sélectif à transistor dont le gain est fixe.

Le modulateur 2 est un amplificateur à transistor dont la tension d'alimentation est commandée par la tension sur l'entrée de modulation. En pratique, sa tension d'alimentation est nulle, ce qui assure le blocage du transistor, ou égale à une tension Vi qui définit la puissance crête du signal délivré par le modulateur.

Le préamplificateur 3 est un amplificateur à transistor chargé par un transformateur de symétrisation.

L'amplificateur de puissance 4 est un amplificateur du type "push-pull" chargé par un transformateur symétrique-dissymétrique.

Le filtre 5 et la tête de traitement 6 peuvent être de conception connue.

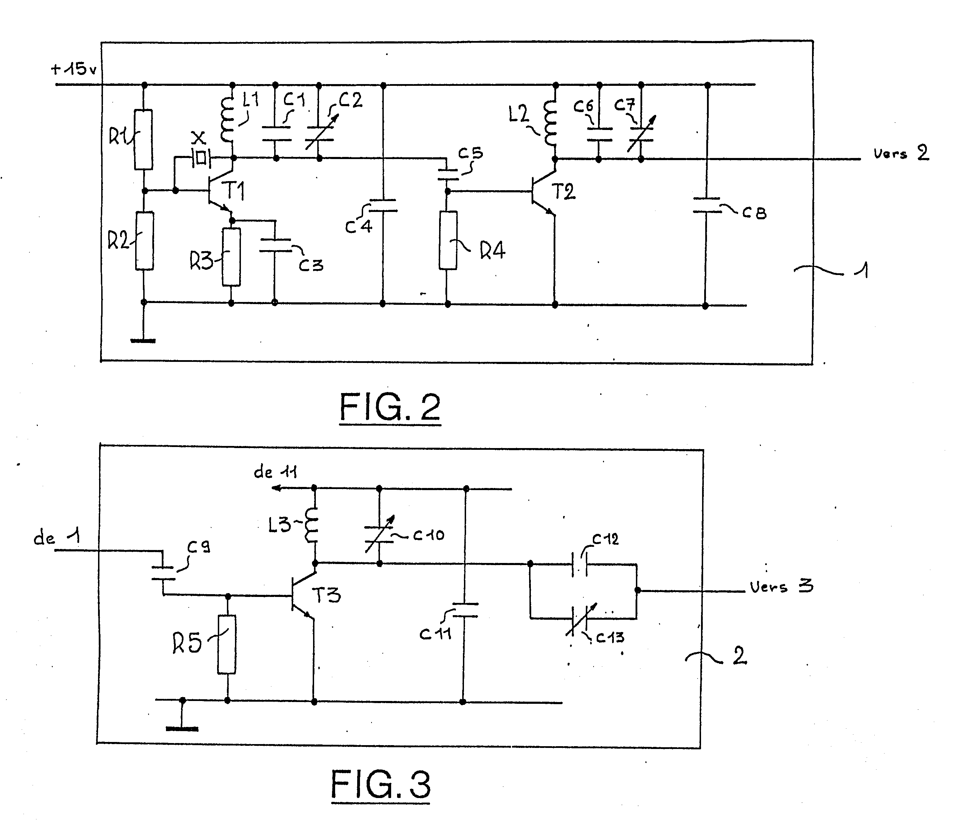

L'oscillateur 1, Fig. 2, comprend un transistor NPN T1 dont la base est reliée au collecteur par un quartz X résonnant à 27,125 MHz et définissant la fréquence des oscillations. La base est reliée au point commun à deux résistances R1 et R2 montées en série entre une source d'alimentation de 15 V et la masse. Le diviseur de tension formé par les résistances R1 et R2 assure sur la base un potentiel de 4,8 V. Le collecteur du transistor T1 est relié à la source de 15 V par un circuit parallèle comprenant une inductance L1, un condensateur fixe C1 et un condensateur variable C2. Les valeurs de L1 et de C1, C2 sont choisies de manière que le montage oscille à la fréquence de 27,125 MHz du quartz X. L'émetteur du transistor T1 est relié à la masse par une résistance R3 et un condensateur C3 en parallèle. La tension de polarisation de l'émetteur est ainsi réglée à 4,2 V. Dans ces conditions de polarisation, le transistor T1 fonctionne en classe A. Un condensateur C4 assure le découplage entre la source de 15 V et la masse.

Le collecteur de T1 est relié par un condensateur de liaison C5 à la base d'un transistor NPN T2, qui constitue le composant actif de l'amplificateur à gain fixe. La base de T2 est encore reliée à la masse par une résistance R4, son émetteur est relié directement à la masse et son collecteur est relié à la source de 15 V par un circuit parallèle comprenant une inductance L2, un condensateur fixe C6 et un condensateur variable C7. La polarisation apportée par la résistance R4 est telle que le transistor T2 fonctionne en classe C. Un condensateur C8 assure le découplage entre la source de + 15 V et la masse.

Le collecteur du transistor T2 délivre le signal à 27,125 MHz fourni par l'oscillateur T1-X et amplifié par T2.

Dans le préamplificateur 3, Fig. 3, la sortie de 2, c'est à dire le collecteur de T2, est reliée, par un condensateur de liaison C9, à la base d'un transistor NPN T3. La base de T3 est reliée à la masse par une résistance R5, son émetteur est relié directement à la masse et son collecteur est relié à une source de tension modulée Vi par un circuit parallèle comprenant une inductance L3 et un condensateur variable C10. Un condensateur C11 assure le découplage entre la source Vi et la masse. La polarisation de la base de T3, assurée par R5, est telle que le transistor T3 fonctionne en classe C.

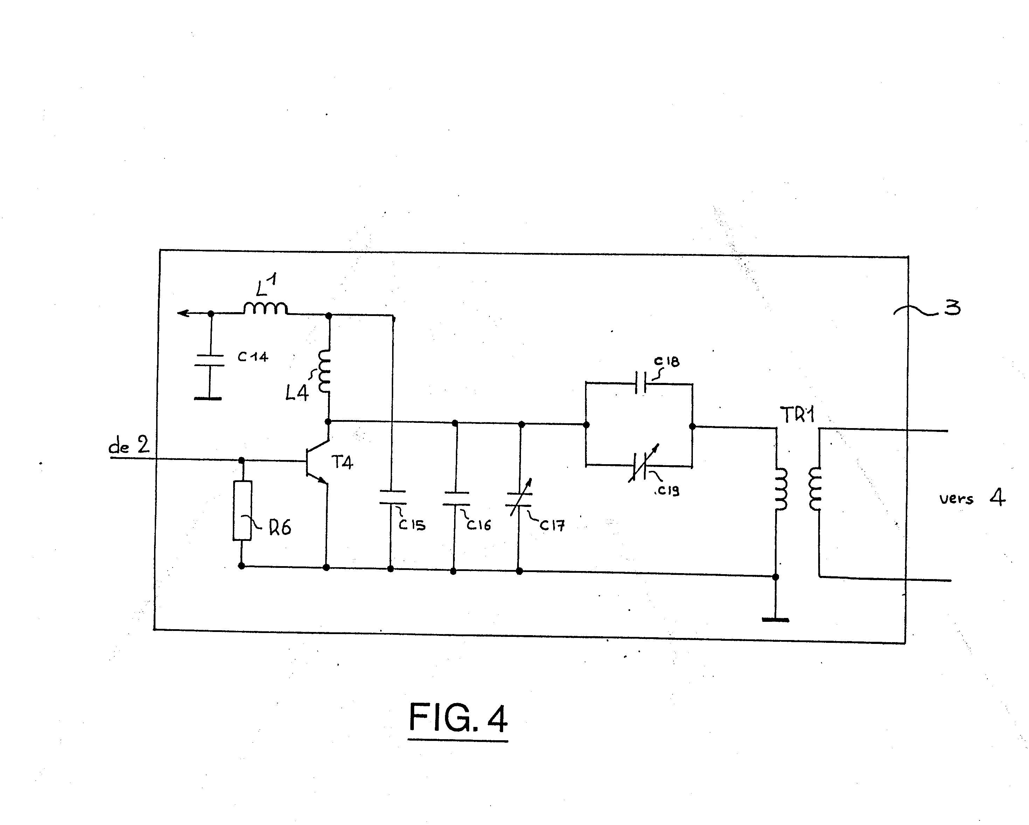

La sortie du préamplificateur 3, c'est à dire le collecteur de T3, est reliée par deux condensateurs C12 et C13 à la base du transistor NPN T4 de l'amplificateur 4, Fig. 4. La base de T4 est reliée à la masse par une résistance R6, son émetteur est relié directement à la masse et son collecteur est relié à une source de 24 V par une inductance L4 et une self de choc $L^1$ dont la borne reliée à la source est reliée à la masse par un condensateur C14. Entre le point commun à $L^1$ et L4, d'une part, et la masse, d'autre part, est monté un condensateur de découplage C15. Le collecteur de T4 est encore relié, d'une part, à la masse par un condensateur fixe C16 et un condensateur variable C17 en parallèle, et, d'autre part, à une borne du primaire d'un transformateur TR1 par un circuit parallèle comprenant un condensateur fixe C18 et un condensateur variable C19. L'autre borne du primaire de TR1 est relié à la masse. Le transformateur TR1 est, de préférence, constitué d'un tore du type 4C6 sur lequel est enroulé le secondaire formé de quatre tours de bande de cuivre de 3 mm de largeur et le primaire formé de huit tours de cuivre de 0,5 mm de diamètre.

La polarisation de T4 est assurée par R6 de manière que ce transistor fonctionne en classe C. Le circuit d'accord parallèle est formé par l'inductance L4 et les condensateurs C16 et C17, tandis qu'un autre circuit de charge du collecteur de T4 est formé par le transformateur TR1 dont l'inductance de fuite est accordée par les condensateur C18, C19.

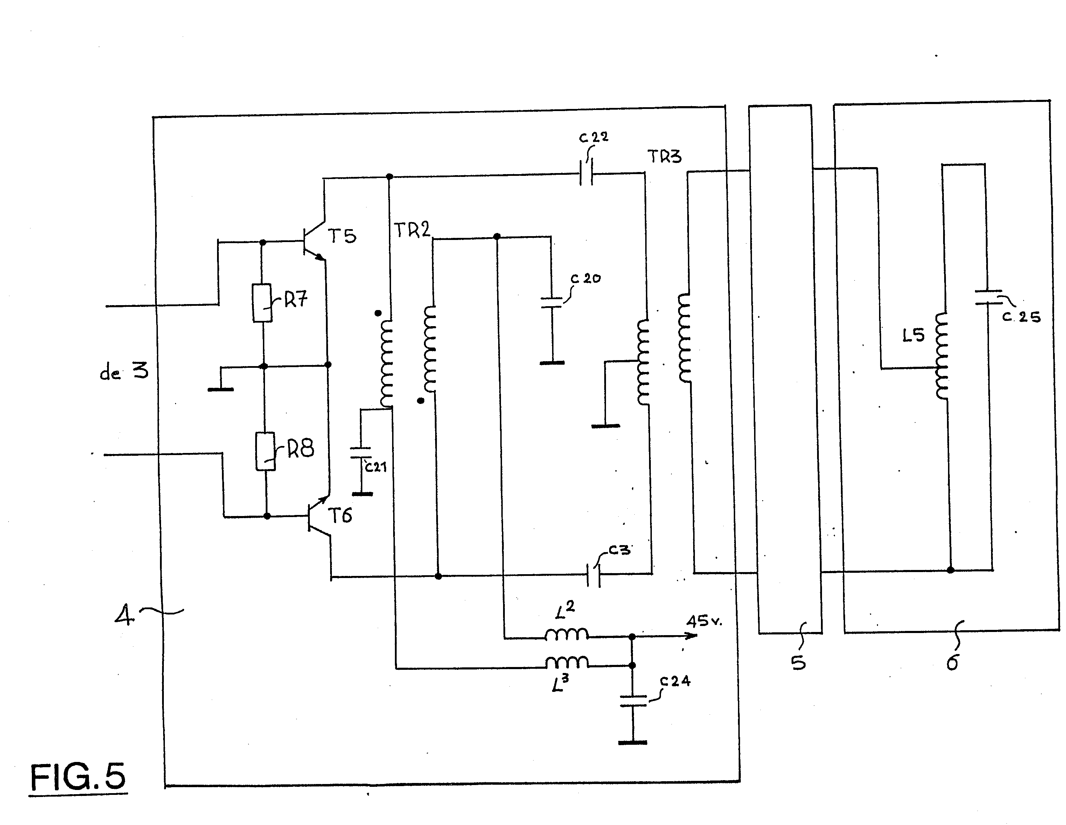

Dans l'amplificateur de puissance 4, Fig. 5, le secondaire du transformateur TR1 a ses bornes respectivement reliées aux bases de deux transistors NPN T5 et T6, montés en "push-pull". Les bases de T5 et T6 sont respectivement reliées à la masse par des résistances R7 et R8. Les émetteurs de T5 et T6 sont reliés en parallèle directement à la masse. Les collecteurs de T5 sont respectivement reliés à une source de 45 V par deux enroulements d'un transformateurs TR2 en série avec deux selfs de chocs $L^2$ et $L^3$. La source de 45 V est découplée à la masse par un condensateur C24. Les enroulements de TR2 sont formés de cinq tours de fils sur un barreau de ferrite commun. Le point commun à un enroulement et à la self $L^2$ est relié à la masse par un condensateur C20 tandis que l'autre point commun à l'autre enroulement et la self $L^3$ est relié à la masse par un condensateur C21. Les collecteurs de T5 et T6 sont encore reliés aux bornes du primaire d'un transformateur TR3 par deux condensateurs de liaison C22 et C23. Le point milieu du primaire de TR3 est à la masse.

Une borne du secondaire du transformateur TR3 est à la masse tandis que l'autre est reliée à l'entrée du filtre 6 dont la sortie est reliée à la tête de traitement 6 qui, du point de vue électrique, se comporte comme un enroulement inductif L5 dont un point intermédiaire est relié à la sortie du filtre, une borne est à la masse et l'autre borne est reliée à l'électrode d'un condensateur C25 dont l'autre électrode est à la masse.

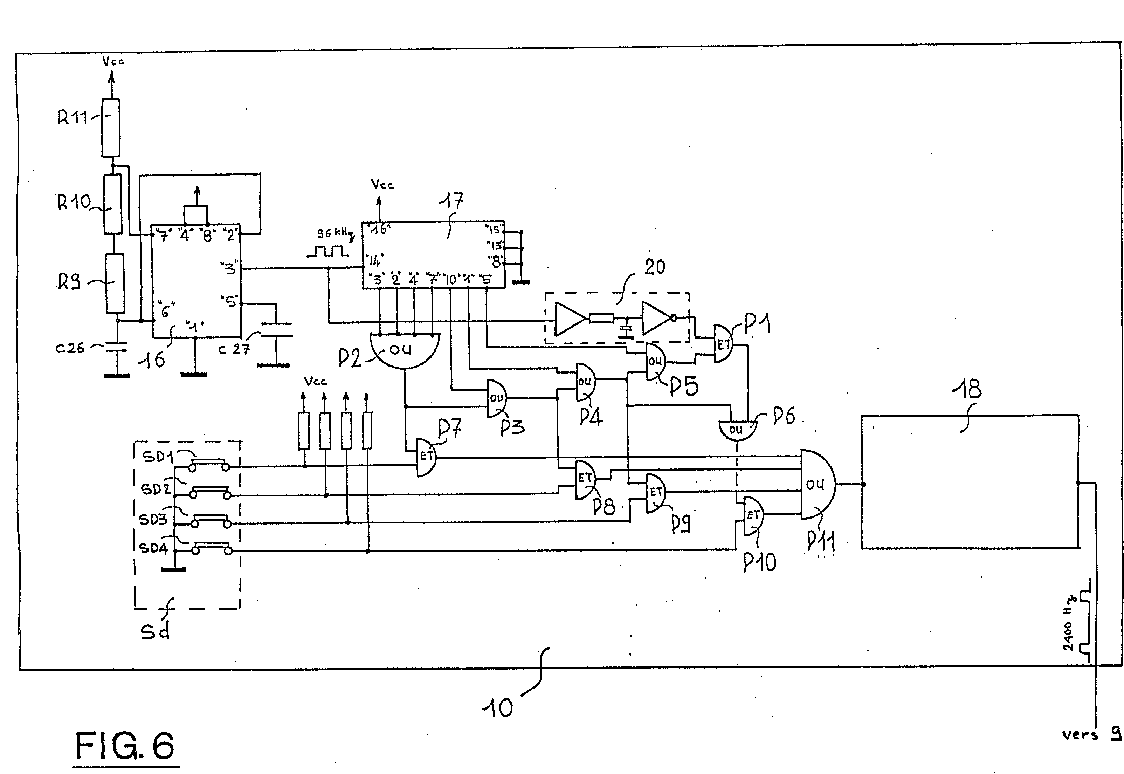

Le circuit de réglage des largeurs des impulsions 10, Fig. 6, comprend un oscillateur 16, un circuit diviseur par dix 17, un circuit diviseur par quatre 18 et un certain nombre de portes ET et OU.

L'oscillateur 16 est un circuit intégré 19, commercialisé sous la référence 555. Le circuit 555 à sa borne "6" reliée à la source de tension Vcc par trois résistances R9, R10 et R11 en série, la résistance R10 étant ajustable. Ses bornes "2" et "6" sont reliées au point commun aux résistances R9 et C26. Ses bornes "6" et "5" sont respectivement découplées de la masse par des condensateurs C26 et C27. Ses bornes "4" et "8" sont reliées à la source + Vcc. Les valeurs choisies pour les composants sont telles que sa borne "3" délivre un signal en impulsions à la fréquence de 96 kHz. L'accord sur la fréquence de 96 kHz est obtenue en choisissant les valeurs des résistances R9 à R11 et de la capacité de C26.

La sortie de 16 est, d'une part, reliée à l'entrée d'horloge du diviseur 17 et, d'autre part, par l'intermédiaire d'un circuit à retard 20 à une entrée d'une porte ET P1. Le diviseur 17 est constitué par un circuit compteur commercialisé sous la référence 4017. L'entrée du diviseur est reliée à la borne "14". Sa borne "16" est reliée à la source + Vcc. Ses bornes "8", "13" et "15" sont à la masse.

Le compteur 17 fonctionne en diviseur par dix et a ses quatres sorties Q0 à Q3 reliées à l'entrée d'une porte OU P2 dont la sortie délivre des impulsions d'une largeur de 40 microsecondes avec une fréquence de répétition de 9,6 kHz. La sortie Q4 est reliée à l'entrée d'une porte OU P3 dont la seconde entrée est reliée à la sortie de P2. La sortie Q5 est reliée à une entrée d'une porte OU P4 dont l'autre entrée est reliée à la sortie de la porte P3. La sortie Q6 est reliée à une entrée d'une porte OU P5 dont l'autre entrée est reliée à la sortie de la porte P4. Les portes P3, P4 et P5 délivrent respectivement des impulsions de largeurs 50, 60 et 70 microsecondes. La sortie de la porte P5 est reliée à la seconde entrée de la porte ET P1. La sortie de la porte P1 est reliée à une entrée d'une porte OU P6 dont l'autre entrée est reliée à la sortie de la porte P4. La porte P6 délivre des impulsions de largeur 65 microsecondes.

Les sorties des portes P2, P3, P4 et P6 sont respectivement reliées aux premières entrées de portes ET P7, P8, P9 et P10. Les secondes entrées des portes P7 à P10, respectivement polarisées par des résistances reliées à la tension +Vcc, sont respectivement reliées à des contacts de repos de touches TC1 à TC4 dont l'ouverture met la seconde entrée correspondante au niveau Vcc. Les sorties des portes P7 à P10 sont respectivement reliées aux quatre entrées d'une porte OU P11 dont la sortie est reliée l'entrée du diviseur par quatre 18 qui délivrent des impulsions à la fréquence de 2400 Hz. Les

0119148

durées des impulsions délivrées par 18 sont de 40, 50, 60 ou 65 microsecondes suivant la touche TC1 à TC4 qui est enfoncée. L'ensemble des touches TC1 à TC4 est rassemblé dans Sd à la Fig. 1.

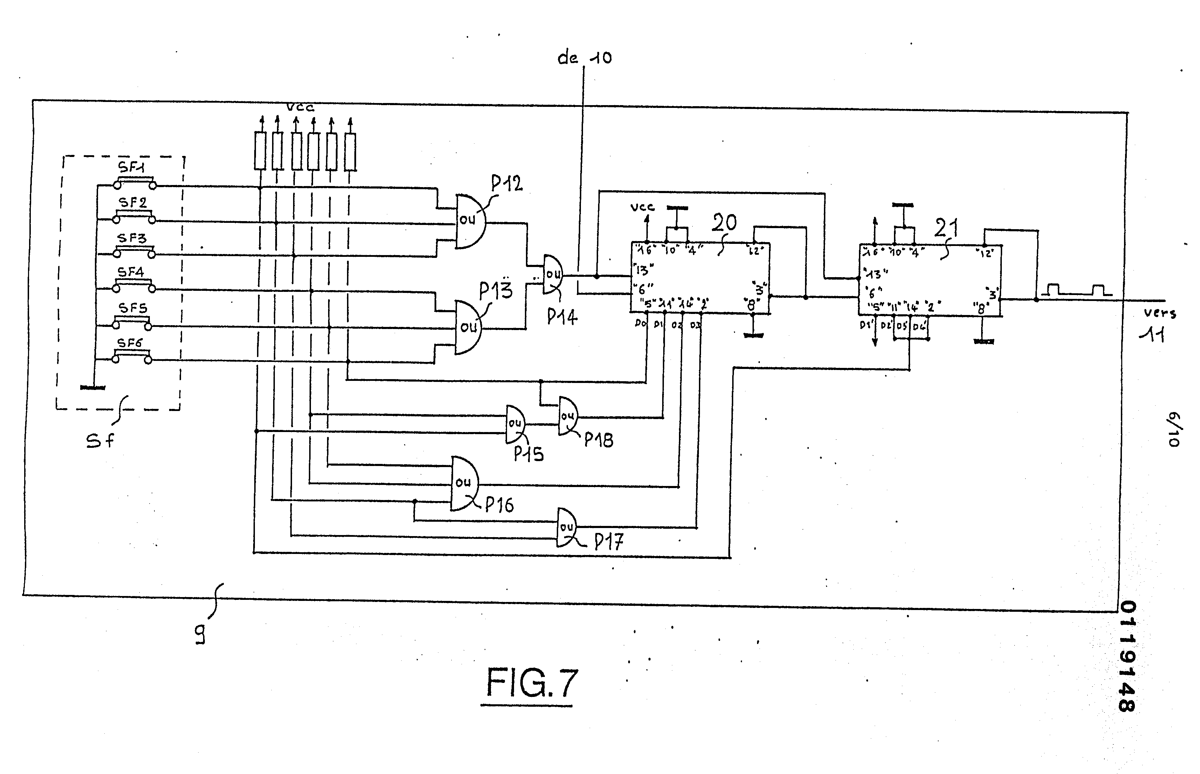

Le circuit 10 de réglage de la fréquence des impulsions, Fig. 7, comprend deux diviseurs 20 et 21 montés en cascade et un ensemble de portes logiques commandées par les contacts des touches Sf. Les diviseurs 20 et 21 sont des circuits compteurs qui sont disponibles dans le commerce sous la référence 4526.

Le diviseur 20 a sa borne "6" d'entrée de signal d'horloge reliée à la sortie du circuit 9 de réglage de largeurs des impulsions qui lui fournit donc un train d'impulsions à 2400 Hz. Sa borne "3" constitue sa sortie de signal qui est reliée, d'une part, à sa propre borne "12" de remise à zéro et, d'autre part, à la borne "6" d'entrée d'horloge du diviseur 21. Ses bornes "4", "8" et "10" sont reliées à la masse et sa borne "16" est reliée à la source de potentiel +Vcc. Ses bornes de commande de rapport de division "5", "11", "14" et "2" forment les entrées D0 à D3.

Le diviseur 21 a sa borne "3" qui délivre le signal de sortie et qui est reliée, d'une part, à sa borne "12" de remise à zéro et, d'autre part, à la cathode d'une diode DI1. Ses bornes "4", "8" et "10" sont reliées à la masse et sa borne "16" est reliée à la source de potentiel +Vcc. Ses bornes de commande de rapport de division "5", "11", "14" et "2" forment les entrées D'0 à D'3.

L'ensemble Sf comprend six touches à contacts de repos SF1 à SF6 qui sont destinées à régler la fréquence de sortie des impulsions de 21 à 80, 200, 300, 400, 600 et 800 Hz, respectivement. Un contact des touches SF1 à SF6 est relié à la masse et l'autre à la source de tension +Vcc par des résistances de polarisation. Les contacts polarisés de SF1 à SF3 et de SF4 à SF6 sont respectivement reliés aux entrées de portes OU P12 et P13 dont les sorties sont reliées aux entrées d'une porte OU P14 dont la sortie est reliée, en parallèle, aux bornes "13" d'activation des diviseurs 20 et 21.

Par ailleurs, le contact polarisé de SF1 est encore relié, d'une part, à une entrée d'une porte OU P15 et, d'autre part, en parallèle aux entrées D'1 à D'3 du diviseur 21. Le contact polarisé de SF2 est encore relié, d'une part, à une entrée d'une porte OU P16 et, d'autre part, à une entrée d'une porte OU P17. Le contact polarisé de SF3 est relié à l'autre entrée de la porte OU P17. Le contact polarisé de SF4 est relié à une entrée de P15 et à une entrée de P16. Le contact polarisé de SF5 est relié à la troisième entrée de P16. Enfin, le contact polarisé de SF6 est relié, d'une part, à une entrée d'une porte OU P18 et, d'autre part, à l'entrée DO du diviseur 20. La sortie de la porte P15 est reliée à la seconde entrée de P18 dont la sortie est reliée à D1. Les sorties de P16 et P17 sont respectivement reliées aux entrées D2 et D3. L'entrée D'0 du diviseur 21 est reliée à la source de potentiel + Vcc.

La table de vérité suivante indique les états des entrées DO à D3 et D'0 à D'3 en fonction des touches SF1 à SF6 actionnées.

Table de vérité

|     | DO | D1 | D2 | D3 | rd | D'0 | D'1 | D'2 | D'3 | rd' | Hz |
|-----|----|----|----|----|-----|-----|-----|-----|-----|-----|-----|
| SF1 | 0 | 1 | 0 | 0 | :2 | 1 | 1 | 1 | 1 | :15 | 80 |
| SF2 | 0 | 0 | 1 | 1 | :12 | 1 | 0 | 0 | 0 | :1 | 200 |
| SF3 | 0 | 0 | 0 | 1 | :8 | 1 | 0 | 0 | 0 | :1 | 300 |
| SF4 | 0 | 1 | 1 | 0 | :6 | 1 | 0 | 0 | 0 | :1 | 400 |
| SF5 | 0 | 0 | 1 | 0 | :4 | 1 | 0 | 0 | 0 | :1 | 600 |
| SF6 | 1 | 1 | 0 | 0 | :3 | 1 | 0 | 0 | 0 | :1 | 800 |

où rd et rd' sont les rapports de division respectifs de 20 et 21.

Il apparaît bien que l'enfoncement d'une des touches SF1 à SF6 permet de sélecter la fréquence des impulsions délivrées par le circuit 10.

Le circuit de réglage de temps de fonctionnement 8 comprend, en cascade, deux monostables 22 et 23 qui sont constitués par des circuits du type 555. La borne "2" de 22 est reliée, d'une part, à la source Vcc par une résistance R12 et, d'autre part, à une armature d'un condensateur de liaison C28. L'autre armature de C28 est reliée à la sortie d'un inverseur I1 dont l'entrée est reliée, d'une part, à la masse par un condensateur C29 et, d'autre part, à la sortie d'une porte ET P19 par l'intermédiaire d'une résistance R13. La borne "1" de 555 est à la masse, sa borne "8" à la tension Vcc et sa borne "5" reliée à la masse par un condensateur C30. Entre la masse et la source Vcc est prévu un circuit RC comprenant un condensateur C31, une résistance fixe R14 et une résistance ajustable R15. Les bornes "6" et "7" sont reliées au point commun au condensateur C31 et à la résistance R14. La borne "3" est reliée par un condensateur de liaison C32 à la borne "2" du circuit 23.

La borne d'entrée "2" de 23 est encore reliée à la source Vcc par une résistance R16. Sa borne "1" est à la masse, sa borne "8" au potentiel Vcc et sa borne "5" reliée à la masse par un condensateur C33. Entre la masse et la source Vcc est prévu un circuit série comprenant un condensateur C34, une résistance fixe R17 et une résistance ajustable R18. Les bornes "6" et "7" sont reliées au point commun à C34 et R17. La borne de sortie "3" est reliée, par un inverseur I2, d'une part, à la cathode d'une diode DI2 et, d'autre part, à une entrée de la porte ET P19.

L'autre entrée de la porte P19 est reliée à la sortie d'une porte ET P20. La sortie de P20 est encore reliée aux bornes "4" de 22 et 23. Les deux entrées de la porte 20 sont respectivement reliées, par des inverseurs I3 et I4, aux contacts de travail de deux touches SM1 et SM2 formant l'ensemble Sm. En pratique, les touches sont montées entre la masse et la source Vcc, par des résistances. Enfin, l'inverseur I3 relié au contact SM1 est reliée à une entrée d'une porte P21.

Le contact SM1 est, en pratique, actionné par un dispositif d'horlogerie qui le maintient fermé pendant la durée complète du traitement, cette durée étant directement réglable sur le dispositif d'horlogerie et pouvant aller de quelques minutes à, par exemple, trente minutes.

Le contact SM2 est celui d'une simple touche.

Les diodes DI1 et DI2 dont les anodes sont reliées à la source Vcc par une résistance R19 forment une porte ET P22. Les cathodes de DI1 et DI2 forment la sortie de P22 et sont reliées à une entrée de la porte ET P21.

Quand l'appareil est sous tension, la sortie de l'inverseur I2 est au niveau haut. Donc, la porte P22 laisse passer le train d'impulsions délivré par le circuit 10. Par ailleurs, quand le dispositif d'horlogerie est mis en marche, l'entrée correspondante de la porte 21 est au niveau haut, si bien que la sortie de 21 délivre le train pendant toute la durée réglée sur le dispositif d'horlogerie. Quand, de plus, on enfonce la touche de cadencement SM2, les circuits 22 et 23 fonctionnent si bien que la sortie de l'inverseur I2 reste au niveau haut pendant un temps T, mais passe au niveau bas pendant un temps T', ce cadencement étant cyclique. Il en résulte alors que la porte 22 est passante pendant le temps T et non passante pendant le temps T'. A titre d'exemple, les réseaux RC de 22 et 23 sont choisis de manière que T = 4 minutes et T' = 2 minutes.

Plus précisément, l'enfoncement verouillé de la touche SM2 rend la porte ET P20 passante, ainsi que la porte ET 19. Donc le monostable 22 fonctionne pendant quatre minutes en appliquant à sa borne "3" un niveau bas et le monostable 23 est au repos. Quand, après quatre minutes, la borne "3" passe au niveau haut, la transition est transmise par C32 à 23 qui applique pendant deux minutes un niveau haut à sa borne "3", ce qui met la sortie de I2 au niveau bas. Après ces deux minutes, le niveau de "3" change si bien que I2 rend à nouveau P19 passante et la transition est transmise par C28 à 22. Le cycle recommence.

Dans le circuit de réglage de tension de crête 11, la sortie de la porte ET 21 est reliée à la base d'un transistor NPN T7 par une résistance R20. Le collecteur de T7 est relié directement à la source Vcc et son émetteur est relié à la masse par deux résistances R21 et R22 en série. De plus, la base de T7 est reliée aux premiers contacts de travail de cinq touches SC1 à SC5. Les seconds contacts des touches SC1 à SC4 sont respectivement reliés à la masse par quatre résistances R23 à R26. Le second contact de SC5 est isolé. Enfin, entre la base et le collecteur de T7 est monté un condensateur C34. Le point commun aux résistances R21 et R22 constitue la sortie du circuit 11. Ce point est relié par des câbles coaxiaux, d'une part, à l'entrée de modulation de 2 et, d'autre part, à l'entrée du circuit 12.

En pratique, quand la porte ET 21 est passante, sa sortie est au potentiel Vcc, si la touche SC5 (ou aucune touche n'est enfoncée, le transistor T7, monté en suiveur, transmet la tension de crête maximale. Si l'une des touches SC1 à SC4 est enfoncée, la tension sur la base de T7 prend une valeur intermédiaire déterminée par la résistance R20 et l'une des résistances R23 à R25, et le transistor transmet une tension de crête intermédiaire correspondante. Le condensateur C35 et la résistance R21 sont montés pour éviter des oscillations dans le circuit de T7.

L'entrée du circuit 11 est reliée, par une résistance R27 à l'entrée non inverseuse d'un amplificateur opérationnel A1, cette entrée non inverseuse étant reliée à la masse par une résistance R28. Les résistances R27 et R28 forment un diviseur de tension. L'entrée non inverseuse est encore reliée à la source Vcc par une résistance R29, qui assure que la tension appliquée à A1 est positive. La sortie de A1 est reliée, d'une part, à son entrée inverseuse et, d'autre part, à l'anode d'une diode DI3 dont la cathode est reliée, d'une part, à la masse par une résistance R30 et, d'autre part, à l'entrée non inverseuse d'un amplificateur opérationnel A2. L'entrée non inverseuse est encore reliée à la masse par un condensateur C35. Les résistances R30, R31 et le condensateur C36 restituent la tension moyenne du signal de sortie de A1. La sortie de A2 est reliée à son entrée inverseuse, ainsi qu'à l'entrée d'un milliampèremètre 12 par deux résistances R32 et R33 en série.

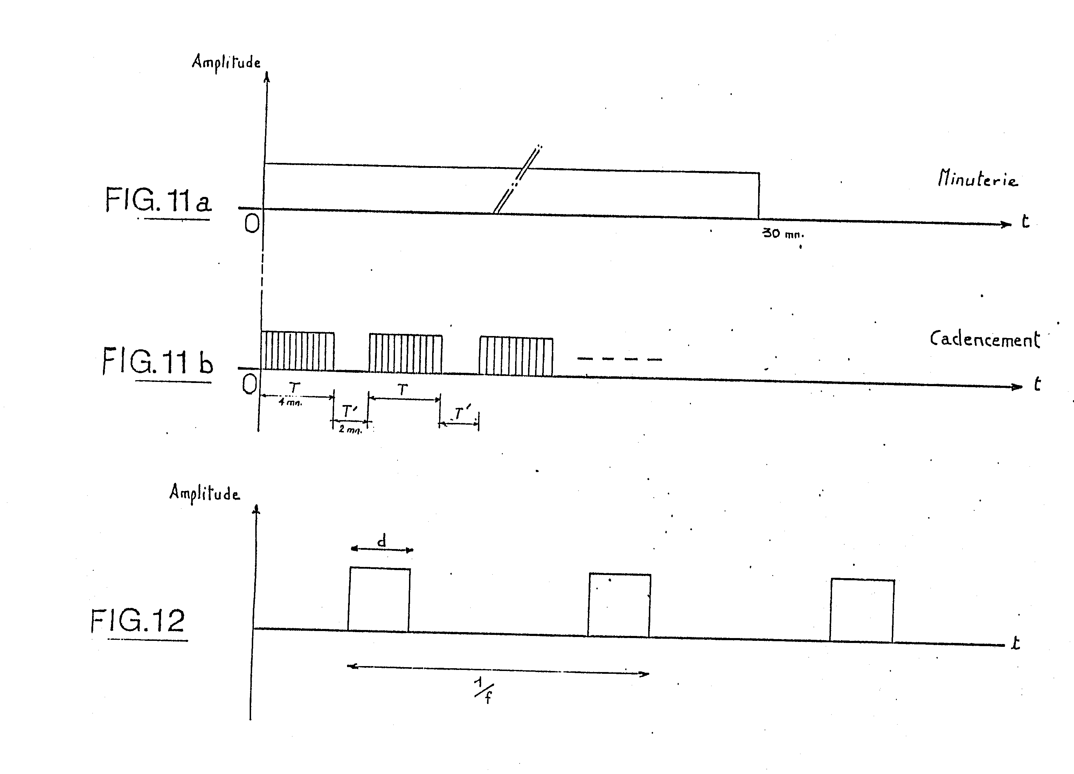

Le diagramme des temps de la Fig. 11A illustre le mode de fonctionnement quand la touche SM2 est enfoncée seule. Pendant une durée, par exemple, de trente minutes, la tête 6 émet un signal haute fréquence à 27,125 MHz dont la puissance de crête est sélectée par les touches Sc, le signal HF étant découpé en impulsions dont la durée est choisie par les touches Sd et la fréquence par les touches Sf.

Le diagramme des temps de la Fig. 11b illustre le mode de fonctionnement cadencé qui est réalisé quand les touches SM1 et SM2 sont actionnées. Dans ce cas, la tête 6 émet toujours un signal HF pulsé dont les paramètres sont choisis comme précédemment, mais l'émission est cadencée avec une période T de travail et une période T' de repos, la durée totale du traitement étant toujours déterminée par la minuterie.

Le diagramme des temps de la Fig. 12 illustre la forme de signal à la sortie du transistor T7. Le signal se compose d'un train d'impulsions de durée d déterminée par Sd, de fréquence déterminée par Sf et d'amplitude déterminée par Sc.

L'appareil de mesure 13 permet au praticien de lire la puissance moyenne du signal émis par la tête de l'appareil et donc de surveiller le traitement effectué.

13                                    **0119148**

REVENDICATIONS

1) Appareil thérapeutique athermique comprenant une partie analogique de génération et de mise en forme du signal haute fréquence émis par la tête de traitement et une partie logique de génération de signaux de découpage temporel et de réglage d'amplitude du signal haute fréquence, caractérisé en ce que la partie analogique comprend, montés en cascade, un oscillateur haute fréquence (1) stabilisé par un quartz (X), un étage de modulation (2), un étage de préamplification (3), un étage d'amplification de puissance (4) et un filtre de bande (5) dont la sortie est reliée à l'entrée d'excitation de la tête (6), et en ce que la partie logique (7) délivre un train d'impulsions appliquées à l'entrée de modulation du modulateur (2) et comprend un circuit numérique de réglage (10) de la durée des impulsions, un circuit numérique de réglage (9) de la fréquence des impulsions, un circuit de réglage numérique (11) de l'amplitude des impulsions et un circuit de réglage (8) de la durée continue ou cadencée du fonctionnement.

2) Appareil suivant la revendication 1, caractérisé en ce que le modulateur (2) est un amplificateur à transistor (T2) fonctionnant en classe C et accordé sur la fréquence de l'oscillateur haute fréquence (1), la tension d'alimentation de l'amplificateur étant définie par la sortie de la partie logique (7) qui lui applique ledit train d'impulsions.

3) Appareil suivant la revendication 1 ou 2, caractérisé en ce que le préamplificateur (3) est un amplificateur à transistor (T3) fonctionnement en classe C dont le circuit de charge comprend un circuit d'accord classique, plus un circuit de charge formé par un transformateur de symétrisation (TR1) dont l'inductance de fuite est accordée par des condensateurs série (C12, C13).

4) Appareil suivant l'une des revendications 1 à 3, caractérisé en ce que l'oscillateur haute fréquence (1) est un oscillateur à transistor (T1), stabilisé par un quartz (X), dont la sortie est reliée par un condensateur (C5) à l'entrée d'un amplificateur accordé à transistor (T2) fonctionnant en classe C dont la tension d'alimentation est fixe, la sortie dudit amplificateur étant reliée à l'entrée de signal HF du modulateur (2).

0119148

5) Appareil suivant l'une des revendications 1 à 4, caractérisé en ce que le circuit de réglage (10) de largeur des impulsions est constitué par un oscillateur basse fréquence (16) délivrant un signal rectangulaire et dont la sortie est reliée à l'entrée d'un compteur (17) dont les sorties sont sélectivement reliées à un jeu de portes OU (P2, P3, P4, P6) délivrant des impulsions de largeurs multiples dudit signal rectangulaire, les sorties des portes OU (P2, P3, P4, P6) étant reliées aux premières entrées d'un jeu de portes ET (P7, P8, P9, P10) dont les autres entrées sont reliées à des touches de sélection (Sd ou SD1 à SD4), les sorties desdites portes ET (P7 à P10) étant réunies par un porte OU (P11) dont la sortie constitue la sortie du circuit de réglage de durée.

6) Appareil suivant l'une des revendications 1 à 5, caractérisé en ce que le circuit numérique de réglage (9) de la fréquence des impulsions est constitué d'un ensemble de diviseurs numériques (20, 21) dont l'entrée de signal est reliée à la sortie du circuit numérique de réglage (10) de durée et dont les rapports de division sont choisies par un jeu de touches (Sf ou SF1 à SF6), la sortie des diviseurs (20, 21) constituant la sortie du circuit de réglage de fréquence (9).

7) Appareil suivant l'une des revendications 1 à 6, caractérisé en ce que le circuit de réglage (11) d'amplitude des impulsions est un étage à transistor (T7) monté en suiveur dont la base est reliée à un point intermédiaire d'un diviseur de tension (R20 et R23 à R26) monté entre la sortie du circuit de réglage de fréquence (9) et la masse, le diviseur de tension étant composé de résistances commutables par un jeu de touches (Sc ou SC1 à SC5).

8) Appareil suivant l'une des revendications 1 à 7, caractérisé en ce qu'entre la sortie du circuit de réglage de fréquence (9) et ledit diviseur de tension (R20 et R23 à R26) est prévu un ensemble de portes ET (P21, P22) dont une entrée est activée pendant des temps définis par le circuit de réglage (8) de la durée continue ou cadencée du fonctionnement.

9) Appareil suivant l'une des revendications 5 à 8, caractérisé en ce qu'entre la sortie de la porte OU de réunion (P11) et la sortie du circuit de réglage (10) de durée des impulsions, est prévu un diviseur de fréquence (18) à rapport fixe.

FIG.1

0119148

0119148

FIG.2

FIG.3

FIG. 4

FIG.5

FIG. 6

0119148

FIG.7

FIG.8

0119148

FIG. 9

0119148

FIG. 10

0119148

Amplitude

FIG. 11 a

Minuterie

O                                    30 mn.                    t

Amplitude

FIG. 11 b

Cadencement

O     T      T'     T      T'                                  t
    4 mn.  2 mn.

Amplitude

FIG. 12

d

1/f                                                           t

10/10

0119148

**0119148**

Office européen
des brevets

Numéro de la demande

EP 84 46 0001

# RAPPORT DE RECHERCHE EUROPEENNE

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. ³) |
|---|---|---|---|
| Y | US-A-4 210 152 (BERRY) <br><br> * Colonne 4, lignes 47-65; colonne 7, lignes 29-53; colonne 10, lignes 22-49; colonne 11, ligne 45 - colonne 12, ligne 49 * | 1,4,5, 8 | A 61 N 1/40 |
| Y | US-A-3 800 802 (BERRY) <br> * Colonne 4, lignes 4-17; colonne 7, ligne 15 - colonne 8, ligne 15; colonne 8, lignes 62-68 * | 1,8 | |
| A | FR-A-2 391 738 (CARBA) <br> * Page 8, ligne 3 - page 10, ligne 10; figures 4,5 * | 1-4,7 | |
| D,A | FR-A-2 097 138 (DIAPULSE) <br> * Page 2, ligne 23 - page 3, ligne 27 * | 1,2 | **DOMAINES TECHNIQUES RECHERCHES (Int. Cl. ³)** |
| A | FR-A-2 394 281 (MEDTRONIC) <br> * Page 4, ligne 5 - page 5, ligne 29 * | 5,6 | A 61 N |
| A | EP-A-0 058 564 (PEARLING) <br> * Page 6, ligne 23 - page 7, ligne 13; page 8, lignes 8-26; figure 6 * | 6,9 | |
| A | FR-A-2 440 202 (ASTIER) | | |

Le présent rapport de recherche a été établi pour toutes les revendications

| Lieu de la recherche <br> LA HAYE | Date d'achèvement de la recherche <br> 06-06-1984 | Examinateur <br> SIMON J.J.E. |
|---|---|---|